# EUROPEAN PATENT SPECIFICATION

(11) **EP 4 494 604 B1**
(45) Date of publication and mention of the grant of the patent: **12.08.2026**
(21) Application number: 24196573.0
(22) Date of filing: 16.07.2021
(51) Int. Cl.: A61F 2/24

(54) **COMMISSURE ASSEMBLIES FORMED FROM TABS OF ASYMMETRIC LEAFLETS**
KOMMISSURANORDNUNGEN AUS LASCHEN ASYMMETRISCHER BLÄTTCHEN
ENSEMBLES DE COMMISSURES FORMÉS À PARTIR DE LANGUETTES DE FEUILLETS ASYMÉTRIQUES

(30) Priority: 17.07.2020 US 202063053176 P
(43) Date of publication of application: 22.01.2025
(62) Document number(s) of the earlier application(s) in accordance with Art. 76 EPC: 21751942.0 / 4 181 829
(73) Proprietor: Edwards Lifesciences Corporation, Irvine, CA 92614 (US)
(72) Inventor: Nir, Noam, Irvine, CA, 92614 (US); Levi, Tamir S., Irvine, CA, 92614 (US)
(74) Representative: Eisenführ Speiser

(56) References cited:
- WO-A1-03/009785
- US-A1- 2020 069 415
- US-B2- 9 717 593

## Description

### FIELD OF THE INVENTION

The present invention relates to implantable prosthetic devices, such as prosthetic heart valves, and to leaflet designs and commissure assembly configurations of such implantable prosthetic devices.

### BACKGROUND OF THE INVENTION

Native heart valves, such as the aortic, pulmonary and mitral valves, function to assure adequate directional flow from, and to, the heart, and between the heart's chambers, to supply blood to the whole cardiovascular system. Various valvular diseases can render the valves ineffective and require replacement with artificial valves. Surgical procedures can be performed to repair or replace a heart valve. Since surgeries are prone to an abundance of clinical complications, alternative less invasive techniques of delivering a prosthetic heart valve over a catheter and implanting it over the native malfunctioning valve have been developed over the years.

Different types of prosthetic heart valves are known to date, including balloon expandable valve, self-expandable valves and mechanically-expandable valves. Different methods of delivery and implantation are also known, and may vary according to the site of implantation and the type of prosthetic valve. One exemplary technique includes utilization of a delivery assembly for delivering a prosthetic valve in a crimped state, from an incision which can be located at the patient's femoral or iliac artery, toward the native malfunctioning valve. Once the prosthetic valve is properly positioned at the desired site of implantation, it can be expanded against the surrounding anatomy, such as an annulus of a native valve, and the delivery assembly can be retrieved thereafter.

A prosthetic valve conventionally includes a circumferential frame, and a leaflet assembly composed of a plurality of leaflets attached to the frame via a plurality of commissure assemblies, and configured to regulate blood flow through the prosthetic valve. There is an ongoing need to provide improved leaflet designs and commissure assembly configurations, for improvement of long-term durability within the patient's body after valve implantation.

WO 03/009785 A1 relates to a method of cutting material for use in an implantable medical device, which employs a plotted laser cutting system. The laser cutting system is computer controlled and includes a laser combined with a motion system. The laser precisely cuts segments out of source material according to a predetermined pattern as designated by the computer. The segments are used in constructing implantable medical devices. The cutting energy of the laser is selected so that the cut edges of the segments are melted to discourage delamination or fraying, but communication of thermal energy into the segment beyond the edge is minimized to avoid damaging the segment adjacent the edge.

US 2020/069415 A1 relates to a method of implanting a prosthetic heart valve including selecting a prosthetic heart valve. The selected prosthetic heart valve has a nominal diameter that is greater than a native annulus diameter of a native annulus by up to forty percent. The method further comprises compressing the selected prosthetic heart valve to a radially compressed configuration in which the selected prosthetic heart valve has a first diameter that is less than the nominal diameter, positioning the selected prosthetic heart valve within the native annulus, and expanding the selected prosthetic heart valve from the radially compressed configuration to a radially expanded configuration in which the selected prosthetic heart valve has a second diameter which is less than the nominal diameter by up to ten percent and which is greater than the first diameter.

US 9,717,593 B2 relates to a collapsible prosthetic heart valve including a collapsible and expandable stent and a collapsible and expandable valve assembly. The stent has a proximal end and a distal end. A plurality of commissure points is disposed on the stent. The valve assembly is disposed within the stent and includes a plurality of leaflets. Each leaflet has a free edge. An end portion of the free edge of each leaflet is folded and sutured to a corresponding one of the plurality of the commissure points.

### SUMMARY OF THE INVENTION

The invention is directed to a leaflet assembly according to independent claim 1 and to a method of assembling a leaflet assembly according to independent claim 11.

The disclosure is directed toward prosthetic heart valves that include an expandable frame and a leaflet assembly mounted therein, wherein the leaflet assembly is coupled to the frame via a plurality of commissure assemblies, formed from tabs of adjacent asymmetrical leaflets secured to each other, wherein at least one of the leaflet tabs includes a vertical tab portion folded over the tab of an adjacent leaflet.

According to the disclosure, there is provided a prosthetic heart valve comprising a frame movable between a radially compressed configuration and a radially expanded configuration, and a leaflet assembly mounted within the frame and comprising a plurality of leaflets configured to regulate flow through the prosthetic valve. Each leaflet comprises a pair of oppositely-directed tabs, wherein each of the tabs comprises a lateral tab portion. At least one of the tabs of each leaflet comprises a vertical tab extension, extending at a non-zero angle from the lateral tab portion. The leaflet is asymmetrical with respect to a leaflet central axis thereof. The at least one vertical tab extension of each leaflet is folded over a lateral tab portion of an adjacent leaflet to form a plurality of annularly spaced commissure assemblies of the leaflet structure.

According to some embodiments, the vertical tab portions of all commissure assemblies are positioned radially inward with respect to the frame.

According to a preferred embodiment of the disclosure, the at least one vertical tab extension is orthogonal with respect to the lateral tab portion it extends from.

According to a preferred embodiment of the disclosure, each leaflet comprises two vertical tab extensions, extending from both lateral tab portions in opposite vertical directions with respect to each other.

According to some embodiments, each vertical tab extension of two adjacent vertical tab extension within each commissure assembly, is folded over a lateral tab portion of an adjacent leaflet of the same commissure assembly.

According to some embodiments, at least one suture is stitched through both vertical tab extensions and both lateral tab portions of each commissure assembly, in an in and out pattern.

According to some embodiments, the length of both vertical tab extensions of each leaflet is identical.

According to some embodiments, the length of the at least one vertical tab extension is at least as great as 50% of the width of the lateral tab portion.

According to some embodiments, the length of the at least one vertical tab extension is at least as great as the width of the lateral tab portion.

According to some embodiments, the length of the at least one vertical tab extension is at least as great as the sum of the width of the lateral tab portion and the thickness of the tab.

According to some embodiments, each leaflet comprises a single vertical tab extension, vertically extending from one of the lateral tab portions.

According to a preferred embodiment of the disclosure, each vertical tab extension within each commissure assembly, is folded over a lateral tab portion of an adjacent leaflet of the same commissure assembly, and folded back over the lateral tab portion from which the vertical tab extension is extending.

According to some embodiments, at least one suture is stitched through both folded portions of the vertical tab extension and both lateral tab portions of each commissure assembly, in an in and out pattern.

According to some embodiments, the length of the at least one vertical tab extension is at least as great as 200% of the width of the lateral tab portion.

According to some embodiments, the length of the at least one vertical tab extension is at least as great as the sum of the 200% of the width of the lateral tab portion and 200% of the thickness of the tab.

According to some embodiments, each leaflet is cut from bovine pericardium.

According to a preferred embodiment of the disclosure, there is provided a leaflet assembly a plurality of leaflets. Each leaflet comprises a pair of oppositely-directed tabs. Each of the tabs comprises a lateral tab portion, wherein at least one of the tabs of each leaflet comprises a vertical tab extension extending at a non-zero angle from the lateral tab portion. Each leaflet is asymmetrical with respect to a leaflet central axis thereof.

According to a preferred embodiment of the disclosure, the at least one vertical tab extension is orthogonal with respect to the lateral tab portion it extends from.

According to some embodiments, each leaflet comprises two vertical tab extensions, extending from both lateral tab portions in opposite vertical directions with respect to each other.

According to some embodiments, each vertical tab extension of two adjacent vertical tab extensions within each commissure assembly, is folded over a lateral tab portion of an adjacent leaflet of the same commissure assembly.

According to some embodiments, at least one suture is stitched through both vertical tab extensions and both lateral tab portions of each commissure assembly, in an in and out pattern.

According to some embodiments, the length of both vertical tab extensions of each leaflet is identical.

According to some embodiments, the length of the at least one vertical tab extension is at least as great as 50% of the width of the lateral tab portion.

According to some embodiments, the length of the at least one vertical tab extension is at least as great as the width of the lateral tab portion.

According to some embodiments, the length of the at least one vertical tab extension is at least as great as the sum of the width of the lateral tab portion and the thickness of the tab.

According to some embodiments, each leaflet comprises a single vertical tab extension, vertically extending from one of the lateral tab portions.

According to some embodiments, each vertical tab extension within each commissure assembly, is folded over a lateral tab portion of an adjacent leaflet of the same commissure assembly, and folded back over the lateral tab portion from which the vertical tab extension is extending.

According to some embodiments, at least one suture is stitched through both folded portions of the vertical tab extension and both lateral tab portions of each commissure assembly, in an in and out pattern.

According to some embodiments, the length of the at least one vertical tab extension is at least as great as 200% of the width of the lateral tab portion.

According to some embodiments, the length of the at least one vertical tab extension is at least as great as the sum of the 200% of the width of the lateral tab portion and 200% of the thickness of the tab.

According to some embodiments, each leaflet is cut from bovine pericardium.

According to yet another aspect of the disclosure, there is provided a method of assembling a leaflet assembly. The method comprises a step of providing a plurality of asymmetrical leaflets, wherein each leaflet comprises a pair of oppositely-directed tabs, wherein each of the tabs comprises a lateral tab portion, and wherein at least one of the tabs of each leaflet comprises a vertical tab extension extending at a non-zero angle from the lateral tab portion.

The method further comprises a step of folding a vertical tab extension of one of the leaflets over a lateral tab portion of an adjacent leaflet to form a plurality of annularly spaced commissure assemblies of the leaflet structure.

According to a preferred embodiment of the disclosure, at least one vertical tab extension is orthogonal with respect to the lateral tab portion it extends from.

According to a preferred embodiment of the disclosure, each leaflet comprises two vertical tab extensions, extending from both lateral tab portions in opposite vertical directions with respect to each other.

According to a preferred embodiment of the disclosure, the step of folding vertical tab extension of one of the leaflets over a lateral tab portion of an adjacent leaflet, comprises folding each vertical tab extension of two adjacent vertical tab extensions within each commissure assembly, over a lateral tab portion of an adjacent leaflet of the same commissure assembly.

According to some embodiments, the method further comprises a step of stitching a suture through both vertical tab extensions and both lateral tab portions of each commissure assembly.

According to some embodiments, the stitching is performed in an in and out pattern.

According to some embodiments, the length of both vertical tab extensions of each leaflet is identical.

According to some embodiments, the length of the at least one vertical tab extension is at least as great as 50% of the width of the lateral tab portion.

According to some embodiments, the length of the at least one vertical tab extension is at least as great as the width of the lateral tab portion.

According to some embodiments, the length of the at least one vertical tab extension is at least as great as the sum of the width of the lateral tab portion and the thickness of the tab.

According to some embodiments, each leaflet comprises a single vertical tab extension, vertically extending from one of the lateral tab portions.

According to the preferred embodiment of the disclosure, the step of folding vertical tab extension of one of the leaflets over a lateral tab portion of an adjacent leaflet, comprises folding each vertical tab extension within each commissure assembly, a lateral tab portion of an adjacent leaflet of the same commissure assembly, and then folding it back over the lateral tab portion from which the vertical tab extension is extending.

According to some embodiments, the method further comprises a step of stitching a suture through both folded portions of the vertical tab extension and both lateral tab portions of each commissure assembly.

According to some embodiments, the stitching is performed in an in and out pattern.

According to some embodiments, the length of the at least one vertical tab extension is at least as great as 200% of the width of the lateral tab portion.

According to some embodiments, the length of the at least one vertical tab extension is at least as great as the sum of the 200% of the width of the lateral tab portion and 200% of the thickness of the tab.

According to some embodiments, each leaflet is cut from bovine pericardium.

According to the preferred embodiment of the disclosure, the method further comprises a step of mounting the leaflet assembly within a frame of a prosthetic heart valve.

According to some embodiments, the step of mounting comprises positioning the vertical tab portions of all commissure assemblies radially inward with respect to the frame.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the invention will become more apparent from the following detailed description, which proceeds with reference to the accompanying figures.

### BRIEF DESCRIPTION OF THE FIGURES

Some embodiments of the invention are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the invention. For the sake of clarity, some objects depicted in the figures are not to scale.

In the Figures:
Fig 1A. shows a side view of a frame of a prosthetic valve, according to some embodiments not being covered by the present invention.
Fig 1B. shows an enlarged side view of one type of an axially extending window frame portion not being covered by the present invention .
Fig 1C. shows an enlarged side view of another type of an axially extending window frame portion not being covered by the present invention.
Fig. 2 shows a view in perspective of a prosthetic valve, according to some embodiments not being covered by the present invention.
Fig. 3 shows a single leaflet, according to some embodiments not being covered by the present invention.
Fig. 4A shows an enlarged view in perspective of a commissure assembly mounted within a commissure window, according to some embodiments not being covered by the present invention.
Fig. 4B shows cross-sectional view of a commissure assembly mounted within a commissure window, according to some embodiments not being covered by the present invention.
Fig. 5 shows a plan view of a prosthetic valve, according to some embodiments not being covered by the present invention.
Fig. 6 shows a leaflet having two vertical tab extensions, oriented in opposite directions with respect to each other, according to some embodiments .
Fig. 7A shows an enlarged view in perspective of a commissure assembly comprising tabs of adjacent leaflets of the type shown in Fig. 6 secured to each other, according to some embodiments.
Fig. 7B shows a cross sectional view along plane 7B-7B of Fig. 7A.
Fig. 8 shows a leaflet having a single vertical tab extension, according to some embodiments not being covered by the present invention.
Fig. 9A shows an enlarged view in perspective of a commissure assembly comprising tabs of adjacent leaflets of the type shown in Fig. 8 secured to each other, according to some embodiments not being covered by the present invention.
Fig. 9B shows a cross sectional view along plane 9B-9B of Fig. 7A

### DETAILED DESCRIPTION OF SOME EMBODIMENTS

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present, or problems be solved. The technologies from any example can be combined with the technologies described in any one or more of the other examples.

In view of the many possible embodiments to which the principles of the disclosed technology may be applied, it should be recognized that the illustrated embodiments are only preferred examples and should not be taken as limiting the scope of the disclosed technology. Furthermore, well-known features may be omitted or simplified in order not to obscure the disclosure. In order to avoid undue clutter from having too many reference numbers and lead lines on a particular drawing, some components will be introduced via one or more drawings and not explicitly identified in every subsequent drawing that contains that component.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the terms "have" or "includes" means "comprises." As used herein, "and/or" means "and" or "or," as well as "and" and "or".

Directions and other relative references may be used to facilitate discussion of the drawings and principles herein, but are not intended to be limiting. For example, certain terms may be used such as "inner," "outer," "upper," "lower," "inside," "outside,", "top," "bottom," "interior," "exterior," "left," right," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same.

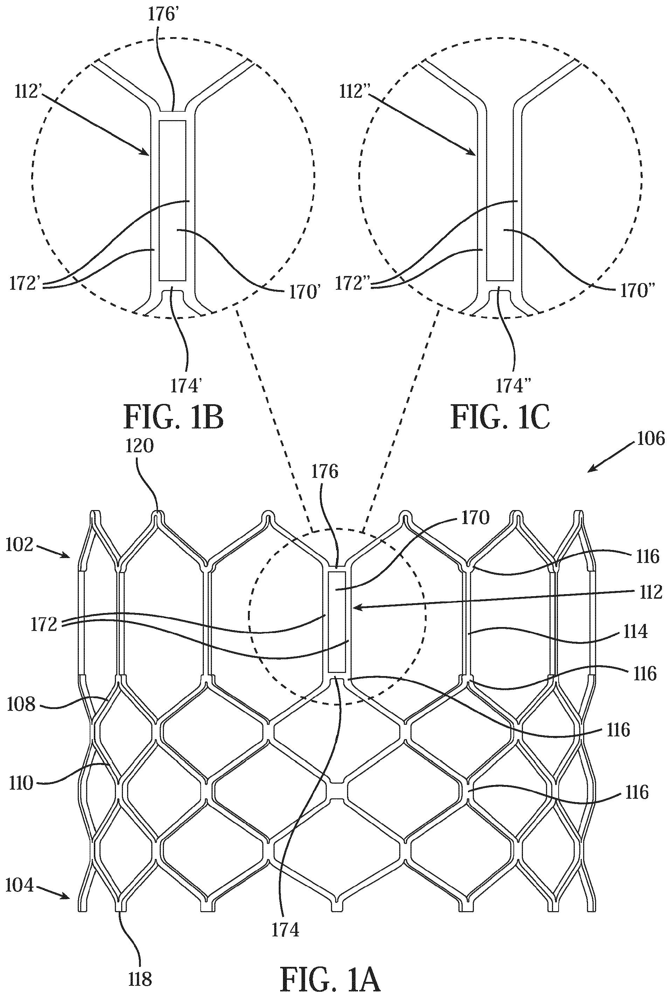

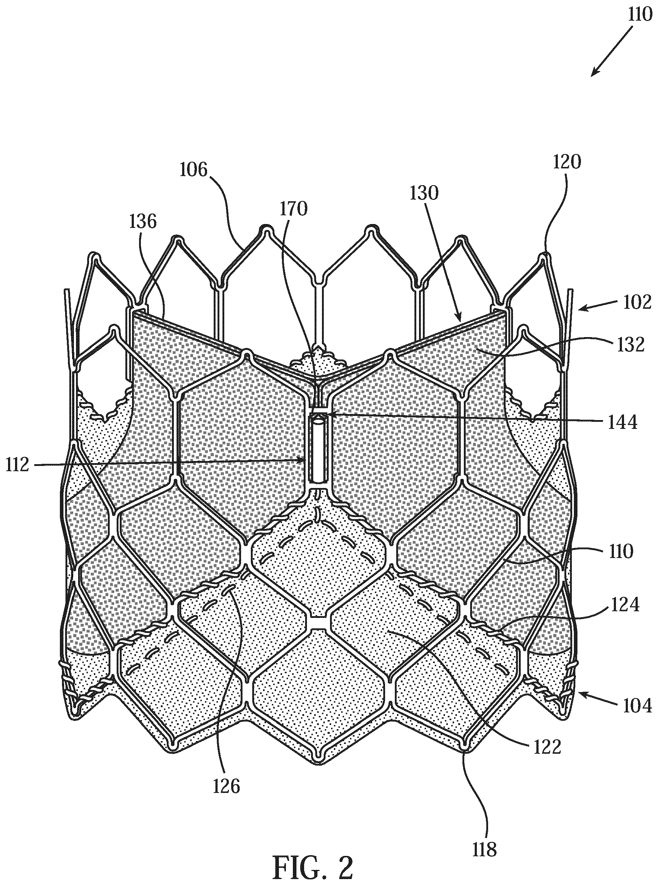

Reference is now made to Figs. 1A-2, showing a prosthetic valve 100, including various components thereof, according to some embodiments. Fig. 1 shows a bare frame 106 of an exemplary prosthetic valve 100. Figs. 1B and 1C show two exemplary types of window frame portions 112 of a prosthetic valve 100. Fig. 2 shows the prosthetic valve 100 of Fig. 1A, including soft components, such as a leaflet assembly 130 and an inner skirt 122, attached to the frame 106.

The prosthetic valve 100 is deliverable to a patient's target site over a catheter (not shown), and is radially expandable and compressible between a radially compressed, or crimped, state, and a radially expanded state - shown in Figs. 1A and 2. The expanded state may include a range of diameters to which the valve 100 may expand, between the compressed state and a maximal diameter reached at a fully expanded state. Thus, a plurality of partially expanded states may relate to any expansion diameter between radially compressed or crimped state, and maximally expanded state. A prosthetic valve 100 of the current disclosure may include any prosthetic valve configured to be mounted within the native aortic valve, the native mitral valve, the native pulmonary valve, and the native tricuspid valve.

The term "plurality", as used herein, means more than one.

As stated above, a prosthetic valve 100 can be delivered to the site of implantation via a delivery assembly carrying the valve 100 in a radially compressed or crimped state, toward the target site, to be mounted against the native anatomy, by expanding the valve 100 via various expansion mechanisms. Fig. 1 shows an exemplary frame 106 of a balloon expandable valve. Balloon expandable valves generally involve a procedure of inflating a balloon within a prosthetic valve, thereby expanding the prosthetic valve 100 within the desired implantation site. Once the valve is sufficiently expanded, the balloon is deflated and retrieved along with the delivery apparatus.

Other types of valves may include other expansion mechanisms, such as mechanical expansion mechanisms or self-expandable mechanisms (not shown). Mechanically expandable valves are a category of prosthetic valves that rely on a mechanical actuation mechanism for expansion. The mechanical actuation mechanism usually includes a plurality of expansions and locking assemblies, releasably coupled to respective actuation assemblies of a delivery apparatus, controlled via a handle for actuating the actuation assemblies to expand the prosthetic valve to a desired diameter. The expansions and locking assemblies may optionally lock the valve's position to prevent undesired recompression thereof, and disconnection of the actuation assemblies from the expansions and locking assemblies, to enable retrieval of the delivery apparatus once the prosthetic valve is properly positioned at the desired site of implantation.

Self-expandable valves include a frame that is shape-set to automatically expand as soon as an outer retaining structure, such as a capsule or a portion of a shaft, is withdrawn proximally relative to the prosthetic valve.

A prosthetic valve 100 can comprise an inflow end 104 and an outflow end portion 102. In some instances, the outflow end 102 is the distal end of the prosthetic valve 100, and the inflow end 104 is the proximal end of the prosthetic valve 100. Alternatively, depending for example on the delivery approach of the valve, the outflow end can be the proximal end of the prosthetic valve, and the inflow end can be the distal end of the prosthetic valve.

The term "proximal", as used herein, generally refers to a position, direction, or portion of any device or a component of a device, which is closer to the user and further away from the implantation site.

The term "distal", as used herein, generally refers to a position, direction, or portion of any device or a component of a device, which is further away from the user and closer to the implantation site.

The term "outflow", as used herein, refers to a region of the prosthetic valve through which the blood flows through and out of the valve 100.

The term "inflow", as used herein, refers to a region of the prosthetic valve through which the blood flows into the valve 100.

The valve 100 comprises an annular frame 106 movable between a radially compressed configuration and a radially expanded configuration, and a leaflet assembly 130 mounted within the frame 106. The frame 106 can be made of various suitable materials, including plastically-deformable materials such as, but not limited to, stainless steel, a nickel based alloy (e.g., a cobalt-chromium or a nickel-cobalt-chromium alloy such as MP35N alloy), polymers, or combinations thereof. When constructed of a plastically-deformable materials, the frame 106 can be crimped to a radially compressed state on a delivery shaft (not shown), and then expanded inside a patient by an inflatable balloon or equivalent expansion mechanisms. Alternatively or additionally, the frame can be made of shape-memory materials such as, but not limited to, nickel titanium alloy (e.g., Nitinol). When constructed of a shape-memory material, such as the case for mechanically expandable valves, the frame can be crimped to a radially compressed state and restrained in the compressed state by insertion into a shaft or equivalent mechanism of a delivery apparatus (not shown).

In the examples illustrated in Figs. 1A and 2, the frame 106 is an annular, stent-like structure comprising a plurality of intersecting struts 110. In this application, the term "strut" encompasses vertical struts, angled struts, attachment posts, commissure windows, and any similar structures described by U.S. Pat. Nos. 7,993,394 and 9,393,110. A strut 110 may be any elongated member or portion of the frame 106. The frame 106 can have one or more multiple rows of cells 108 defined by intersecting struts 110. The frame 106 can have a cylindrical or substantially cylindrical shape having a constant diameter from the inflow end portion 104 to the outflow end portion 102 of the frame as shown, or the frame can vary in diameter along the height of the frame, as disclosed in US Pat. No. 9,155,619.

The end portions of the struts 110 are forming outflow apices 120 at the outflow end 102 and inflow apices 118 at the inflow end 104. The struts 110 can intersect at additional junctions 116 formed between the outflow apices 120 and the inflow apices 118. The junctions 116 can be equally or unequally spaced apart from each other, and/or from the apices 118, 120, between the outflow end 102 and the inflow end 104.

According to some embodiments, the struts 110 include a plurality of angled struts and vertical struts. In the exemplary embodiment illustrated in Fig. 1A, the frame 106 comprises a plurality of angled struts 110, and axially extending struts 114. The struts may be pivotable or bendable relative to each other, so as to permit frame expansion or compression. In some implementations, the frame 106 can be formed from a single piece of material, such as a metal tube, via various processes such as, but not limited to, laser cutting, electroforming, and/or physical vapor deposition, while retaining the ability to collapse/expand radially.

The leaflet assembly 130 comprises a plurality of leaflets 132 (e.g., three leaflets), positioned at least partially within the frame 106, and configured to regulate flow of blood through the prosthetic valve 100 from the inflow end 104 to the outflow end 102. While three leaflets 132 arranged to collapse in a tricuspid arrangement, are shown in the exemplary embodiment illustrated in Figs. 2, it will be clear that a prosthetic valve 100 can include any other number of leaflets 132. The leaflets 132 are made of a flexible material, derived from biological materials (e.g., bovine pericardium or pericardium from other sources), biocompatible synthetic materials, or other suitable materials as known in the art and described, for example, in U.S. Pat. Nos. 6,730,118, 6,767,362 and 6,908,481.

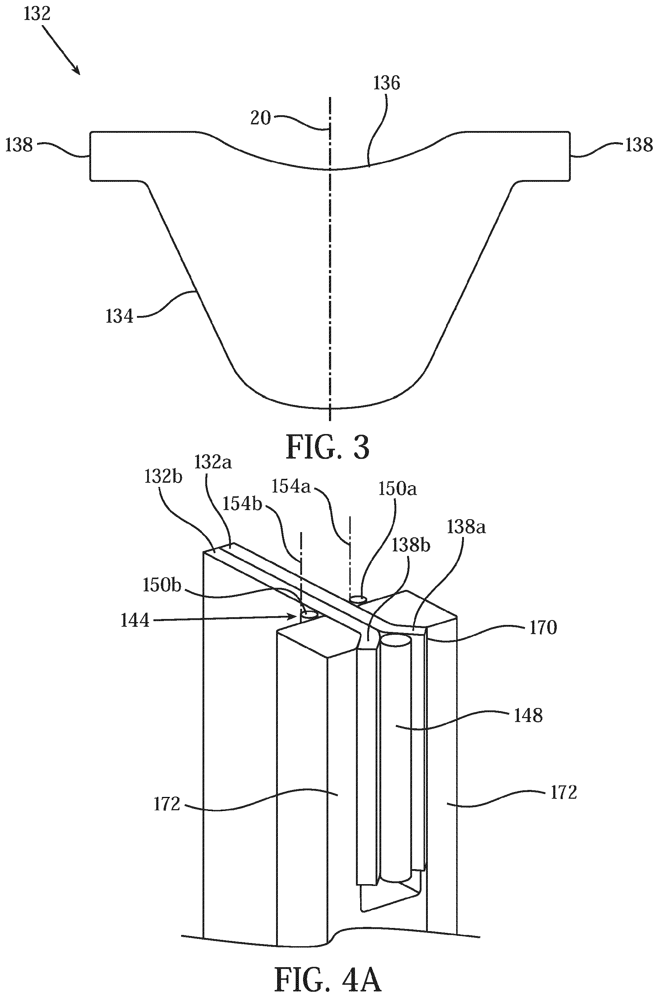

Fig. 3 shows a single representative leaflet 132 in a flattened configuration. The leaflet 132 has a rounded cusp edge 134, and a pair of generally oppositely-directed tabs 138 separating the cusp edge 134 and the free edge 136. The cusp edge 134 forms a single scallop. When the leaflets 132 are coupled to the frame and to each other, the lower edge of the resulting leaflet assembly 130 desirably has an undulating, curved scalloped shape. By forming the leaflets with this scalloped geometry, stresses on the leaflets 132 are reduced which, in turn, improves durability of the valve. Moreover, by virtue of the scalloped shape, folds and ripples at the belly of each leaflet (132), which can cause early calcification in those areas, can be eliminated or at least minimized. The scalloped geometry also reduces the amount of tissue material used to form the leaflet assembly, thereby allowing a smaller, more even crimped profile at the inflow end of the valve.

A conventional leaflet, such as leaflet 132 shown in Fig. 3, is symmetrical with respect to a leaflet central axis 20 extending vertically along it's center, such that if the leaflet 132 is theoretically folded over itself along the leaflet central axis 20, both tabs 138 are congruent with respect to each other.

The leaflets 132 define a non-planar coaptation plane (not annotated) when their free edges 136 co-apt with each other to seal blood flow through the prosthetic valve 100. In some implementations, the free edge 136 of a leaflet 132 can extend along a straight line between the tabs 138. In other implementations, as shown in Fig. 3, the free edge 136 between the tabs 138 is concave with either one or more curvatures (i.e., simple or complex curves).

Leaflets 132 can be secured to one another at their tabs 138 to form commissure assemblies 144 (see, for example, Fig. 4A) of the leaflet assembly 130, which can be secured, directly or indirectly, to structural elements connected to the frame 106 or formed therein, such as commissure posts (not shown) or axially extending window frame portion 112. When secured to two other leaflets 132 to form leaflet assembly 130, the cusp edges 134 of the leaflets 132 collectively form the scalloped shaped lower edge portion of the leaflet assembly 130, which may be coupled, directly or indirectly, to the frame 106, via a scallop-line suture 126 (see Fig. 2).

According to some embodiments, the upper row of cells comprises a plurality of axially extending window frame portions 112 which define commissure windows 170, and a plurality of axially extending struts 114. Each axial strut 114 and each window frame portion 112 extends from a junction 116 defined by the convergence of the lower ends of two angled struts 110 to another junction 116 defined by the convergence of the upper ends of two angled struts 110". Each commissure window frame portion 112 mounts a respective commissure assembly 144 assembly of the leaflet assembly 130.

Figs. 1B and 1C show enlarged views of two exemplary two types of window frame portions 112. It will be appreciated that reference numerals with prime marks (') or double prime marks (") are used to denote different embodiments of the same elements. Embodiments of the disclosed devices and systems may include any combination of different embodiments of the same elements. Specifically, any reference to an element without single or double prime marks may refer to any alternative embodiment of the same element denoted with a single prime mark or double prime marks.

Fig. 1B shows an exemplary window frame portion 112' defining a closed commissure window 170', wherein all reference numerals with prime marks (') shown in Fig. 1B refer to like elements comprised within such a window frame portion 112'. The commissure window 170' is enclosed between a base strut 174' at its bottom, two lateral struts 172' extending axially from the base strut 174'. And an outflow lateral strut 176' opposite to, and parallel with, the base strut 174'. A commissure assembly 144 may be coupled to a window frame portion 112' in such a manner that portions of the commissure assembly 144, such as leaflet tabs 138, extend through the closed window 170'. This may be achieved, for example, by placement of the leaflet assembly 130 within the inner space defined by the frame (106), and extending the tabs 138 radially outward through the closed window 170'.

Fig. 1C shows an exemplary window frame portion 112" defining a closed commissure window 170", wherein all reference numerals with double prime marks (") shown in Fig. 1C refer to like elements comprised within such a window frame portion 112". The commissure window 170" is defined between a base strut 174" at its bottom, and two lateral struts 172" extending axially from the base strut 174". However, unlike the window frame portion 112', the window frame portion 112" does not include an outflow lateral strut (176), thus forming an open-ended commissure window 170". This configuration advantageously allows commissure assemblies (144) to be pre-assembled prior to mounting the commissure assembly within the commissure window 170", which advantageously simplifies the assembly process.

In the illustrated embodiment, each commissure window 170" is substantially U-shaped. However, in other embodiments, the commissure window (170) can have any of various shapes configured to accept the leaflets 132. For example, the commissure windows (170) can be ovular, square, rectangular, triangular, L-shaped, T-shaped, etc.

Additional frame configurations may include commissure posts attached to the frame, configured to accept commissures 144 extending either through window portions defined therein, or supporting commissure attachment thereto in various other manners. Alternatively, some of the cells 108 may be configured to receive commissure assemblies 144 attached thereto. For example, the uppermost row of cells 108 can be configured to receive tabs 138 of the leaflets 132. Further details regarding prosthetic valves, including the manner in which commissures may be mounted to their frames, are described in U.S. Patent Nos. 6,730,118, 7,393,360, 7,510,575, 7,993,394, 8,252,202, and 9,393,110; U.S. Publication Nos. 2018/0325665, 2019/0105153, U.S. Application Nos. 62/869,948 and 62/813,643; and PCT Application No. PCT/US2019/61392. Any of the techniques and mechanisms disclosed in the prior documents can be used to connect the commissure assemblies 144, directly or indirectly, to the frame 106.

According to some embodiments, the prosthetic valve 100 further comprises an inner skirt 122 that can be secured to the inner surface of the frame 106, for example by sutures 124. The inner skirt 122 is configured to assist in securing the leaflet assembly 130 to the frame 106 and to assist in forming a good seal between the valve (100) and the native annulus by blocking the flow of blood through the open cells 108 of the frame (106) below the cusp edges 134 of the leaflets (132). The leaflets 132 may be sutured to the inner skirt 122 via a scallop-line suture 126 that tracks their lower cusp edges 134, while the inner skirt 122 can be sutured to the frame 106 at locations away from the scallop-line suture 126 so that the inner skirt 122 can be more pliable in that area. This can avoid stress concentrations at the scallop-line suture 126.

According to some embodiments, the prosthetic valve 100 can further comprise an outer skirt (not shown) mounted on the outer surface of the frame 106, configure to function, for example, as a sealing member retained between the frame 106 and the surrounding tissue of the native annulus against which the prosthetic valve 100 is mounted, thereby reducing risk of paravalvular leakage past the prosthetic valve 100. Either one of the inner skirt (122) or the outer skirt can be made of various suitable biocompatible materials, such as, but not limited to, various synthetic materials (e.g., PET) or natural tissue (e.g. bovine pericardium).

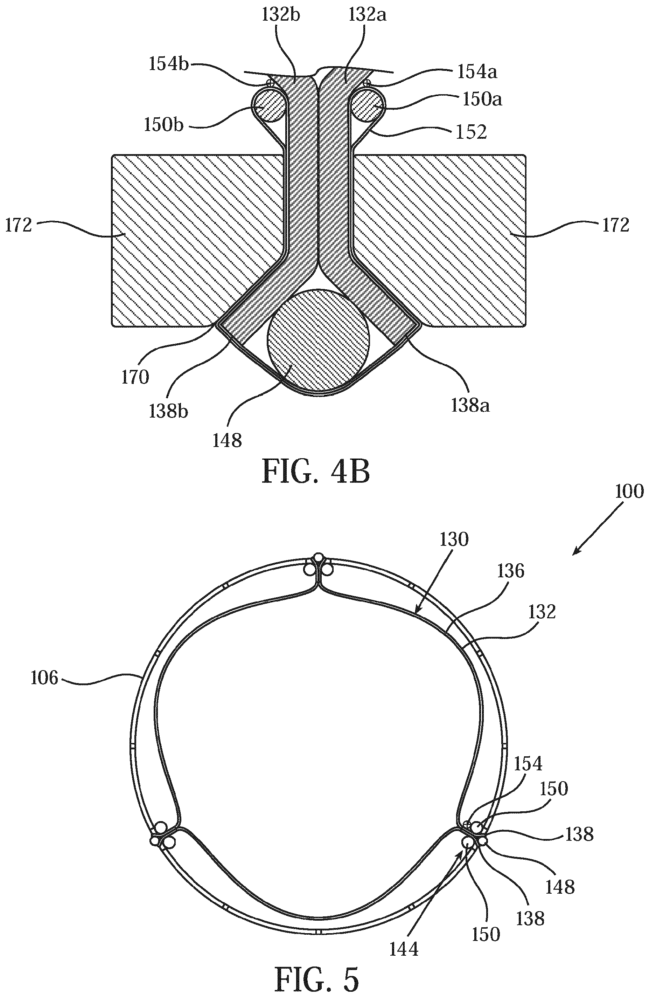

Figs. 4A shows a perspective view of a window frame portion 112 defining a commissure window 170, including a commissure assembly 144 mounter thereto. Fig. 4B shows a cross-sectional view of a commissure assembly 144 mounted within the commissure window 170 of Fig. 4A. As shown, two tabs 138 of leaflets 132 can be inserted into the commissure window 170, either by insertion of the tabs 138 from an inner side of the commissure window 170 to its outer side, such that they may extend radially outward from the commissure window 170, or by sliding both tabs from an upper opening of a commissure window, for example in the case of open commissure windows 170".

A wedge 148 can be inserted between the portions of the tabs 138 that extend through the commissure window 170. The wedge 148 can press portions of the tabs 138 against the outer surfaces of the lateral struts 172. In some implementations, the wedge 148 can be inserted between the tabs 138 after extending the tabs 138 through the commissure window 170. In other implementations, the wedge 148 can be attached first to, and between, the outer portions of the tabs 138, followed by sliding the pre-assembled commissure assembly 144 into the commissure window from an upper opening thereof, for example in the case of an open commissure window 170".

According to some embodiments, as shown, the wedge 148 is an elongated member having a circular shape in cross-section. However, in other embodiments, the wedge can have any of various shapes in cross-section, such as, for example, triangular, ovular, square, rectangular, C-shaped, semi-circular, etc. The wedge 148 can be provided in the form of a wire, chord, sleeve, fabric or suture.

As shown in Figs. 4A-4B, each lateral strut 172 can comprise one or more chamfered surfaces. In the illustrated embodiment, each lateral strut 172 comprises a chamfered surface angled radially outwardly, which may help in securing the leaflets 132 within the commissure window (170). In such configurations, the wedge 148 presses portions of the tabs 138 against the chamfered surfaces. While not shown, it will be clear that each lateral strut (172) can similarly include a chamfered surface angled radially inwardly, which can assist in mitigating frictional contact between the leaflets (132) and the lateral struts 172 during systole. Furthermore, it is to be understood that the lateral struts 172 can be rounded instead of chamfered, to achieve the same potential advantages.

A commissure assembly 144 may further include reinforcement members 150 disposed vertically along the inner side of the window frame portion (112), on both sides of the leaflets 132, such as on both sides of the inner portions of the tabs 138. The reinforcement members 150 are more resistant to bending, or articulating, than the portions of the leaflets 132 that are radially inward of the tabs 138, and can be provided in the form of wires, chords, sleeves, fabrics or sutures.

As shown in Fig. 4B, a fabric strip or cloth 152 can be used to interconnect pairs of adjacent tabs 138 with the reinforcement members 150 and the wedge 148 (cloth removed from view in Fig. 4A for clarity). Cloth 152 can be made from a piece of woven PET fabric, although other synthetic and/or natural materials can be used.

The commissure assembly 144 can then be coupled to window frame portion (112) using, for example, one or more sutures. Further details regarding various attachments techniques and mechanisms for attaching commissures to expansion and locking mechanisms are disclosed in U.S. Publication No. 2018/0325665; U.S. Publication No. 2019/0105153; U.S. Application No. 62/869,948; U.S. Application No. 62/813,643; and PCT Application No. PCT/US2019/61392. Any of the techniques and mechanisms disclosed in the prior documents can be used to connect the commissure assemblies (144) formed by tabs (138) to an axially extending window frame portion (112), to a commissure post attachable to the frame, to commissure clamps attachable to the frame, and/or to commissure windows formed within expansion and locking assemblies of mechanically expandable valves.

During valve cycling, the leaflets can articulate about articulation axes 154, between an open state during systole and coaptation state during diastole. Placement of reinforcement members 150 at the interface of the tabs 138 and the inner surface of the frame 106, helps space the articulation axes 154 away from the frame 106 during normal operation of the prosthetic valve, as further shown in Fig. 5. By having the leaflets 132 flex against reinforcement members 150, contact between the moving portions of the leaflets 132 (especially the free edges 136 of the leaflets) and the frame 106 can be avoided during working cycles, which in turn improves the durability of the valve.

Despite the advantages of utilizing offsetting structures attached to the leaflets 132, such as reinforcement members (e.g., reinforcement members 150) and/or fabric strips (e.g., cloth 152), to space the articulation axes 154 of the leaflets radially inward, with respect to the frame 106, the moving sections of the leaflets 132 may be subject to wear due to abrasion of against such materials.

According to the invention, each leaflet is provided as an asymmetrical leaflet having at least one vertical tab extension, extending at a non-zero angle, preferably orthogonal to, a lateral portion of at least one tab. The at least one vertical tab extension of one leaflet is folded over a lateral tab portion of an adjacent leaflet, and attached thereto, to form a commissure assembly.

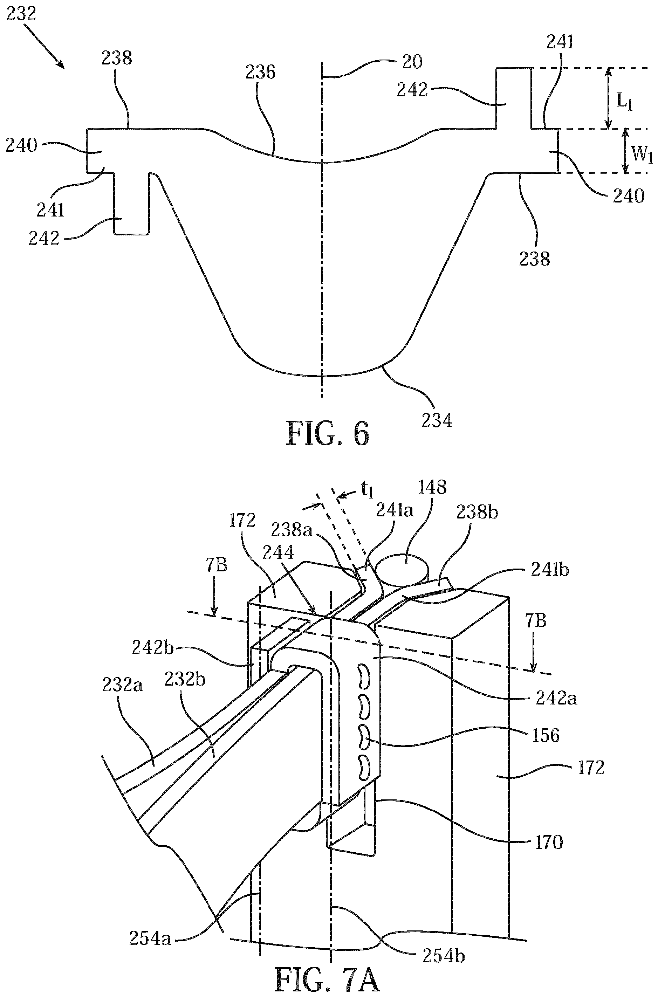

Reference is now made to Figs. 6-7B, showing a preferred embodiment of commissures 244 formed from tabs 238 of leaflets 232. Fig. 6 shows a flattened view of one exemplary type of leaflet 232, which can be similar to leaflet 132, except when noted. The leaflet 232 has a rounded cusp edge 234, and a pair of generally oppositely-directed tabs 238 separating the cusp edge 234 and the free edge 236. Tabs 238 differ from tabs 138 in that each of the tabs 238 further comprises a lateral tab portion 240, which extends in a lateral direction away from the cusp edge 234 and the free edge 236, and a vertical tab extension 242 extending vertically from the corresponding lateral tab portion 240. Each lateral tab portion 240 comprises a lateral outer portion 241, defined as the sub-portion of the lateral tab portion 240 projecting away from the vertical tab extension 242, and having a non-zero length.

Unlike the case with most conventional leaflets, such as leaflet 132 shown in Fig. 3, the leaflet 232 is asymmetrical with respect to a leaflet central axis 20, since the vertical tab extensions 242 on both sides of the leaflet central axis 20 extend in opposite vertical directions with respect to each other. In other words, if the leaflet 232 is theoretically folded over itself along the leaflet central axis 20, both vertical tab extensions 242 extend in opposite directions from each other.

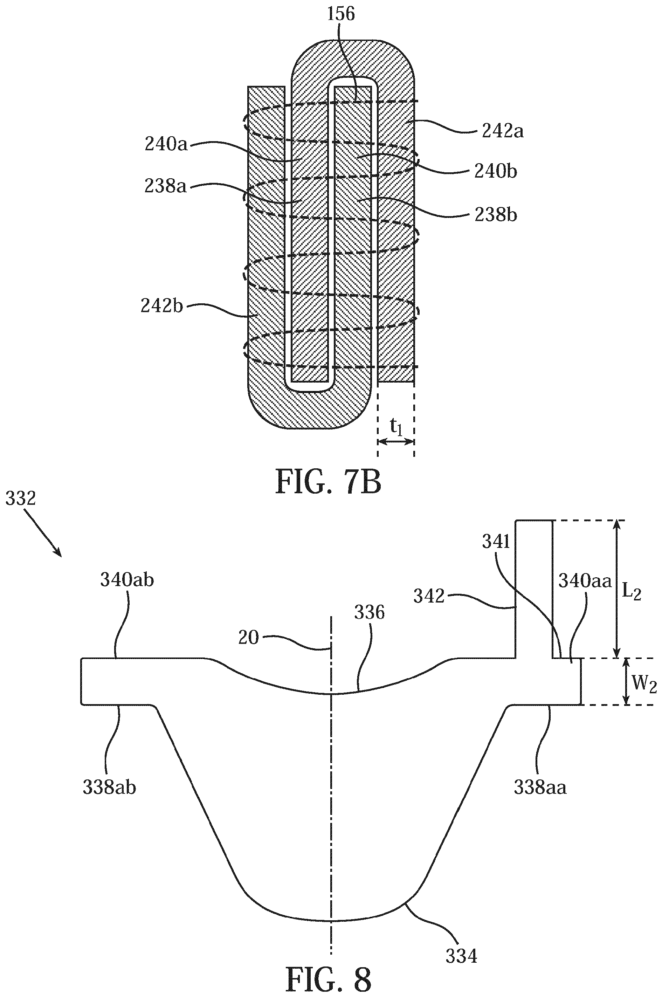

Fig. 7A shows an enlarged view of a commissure 244 mounted within a commissure window 170. Fig. 7B shows a cross-section view of the commissure 244 across plane 7B-7B of Fig. 7A. A commissure 244 is formed by attaching tabs 238 of adjacent leaflets 232 to each other, such that the vertical tab extension 242 of each tab 238 is folded over the adjacent tab 238, and more specifically, over the lateral tab portion 240 of the adjacent tab 238. A suture 156 can be used to couple the adjacent lateral tab portions 240 and the vertical tab extensions 242 folded thereover, to form a four-layered structure as shown in Fig. 7B, extending from the inner surface of the window frame portion 112.

The resulting four layered structure is more resistant to bending, or articulating, than the portions of the leaflets 232 that are radially inward of the tabs 238, and serves to offset the articulation axes 254 radially inward, away from the frame 106. By having the leaflets 232 flex against the inner edges of the vertical tab extensions 242, contact between the moving portions of the leaflets 232 (especially the free edges 236 of the leaflets) and the frame 106 can be avoided during working cycles, which in turn improves the durability of the valve.

According to some embodiments, the leaflets 232, including their tabs 238 with the vertical tab extensions 242, are cut out from bovine pericardium as a single component.

Advantageously, since the vertical tab extensions 242 constitute integral portions of the leaflets 232, the moving portions of the leaflets 232 articulate about portions of the leaflets 232 made of the same material (e.g., bovine pericardium), instead of articulating about external components such as reinforcement members (150) or cloth (152), which can comprise other materials (e.g., textiles, polymeric materials, and the like). The absence of such materials can significantly reduce abrasion and wear of the leaflet material.

As further shown in Fig. 6, each lateral tab portion 240 may have a width W₁, and each vertical tab extension 242 may have a length L₁. Each tab 238 may also have a thickness t₁ (see Fig. 7A). According to some embodiments, the length L₁ of both vertical tab extension 242 on both sides of the leaflet 232 is identical. According to some embodiments, the length L₁ of the vertical tab extension 242 is at least as great as 50% of the width W₁ of the lateral tab portion 240. According to some embodiments, the length L₁ of the vertical tab extension 242 is at least as great as 70% of the width W₁ of the lateral tab portion 240. According to some embodiments, the length L₁ of the vertical tab extension 242 is at least as great as the width W₁ of the lateral tab portion 240. According to some embodiments, the length L₁ of the vertical tab extension 242 is at least as great as the sum of the width W₁ of the lateral tab portion 240 and the thickness t₁ of the tab 238.

As shown in Figs. 7A-7B, two adjacent tabs 238a and 238b of respective leaflets 232a and 232b, may be secured to each other such that the vertical tab extension 242a is folded over lateral tab portion 240b, while the vertical tab extension 242b is folded over lateral tab portion 240a. Since both vertical tab extension 242a and 242b extend from their respective lateral tab portion 240 at opposite directions relative to each other, it is possible to fold them over the adjacent lateral tab portion 240 from opposite sides, thereby preventing any interference therebetween during the folding procedure.

Once in the folded position shown in Figs. 7A-7B, a suture 156 may be used to stitch all four layers, including the vertical tab extension 242a, the lateral tab portion 240b, the lateral tab portion 240a and the vertical tab extension 242b, together. It is noted that suture 156 is used by way of illustration and not limitation, and that other securement means may be used to attach the four layers to each other. Moreover, more than one suture 156 may be used, and while shown to pass through the layers in an in-and-out pattern, any other stitching pattern is contemplated.

As shown in Fig. 7A, the folded vertical tab extensions 242 of the commissure assembly 244 are positioned radially inward with respect to the frame 106. The remaining portions of the lateral tab portions 240, extending beyond the vertical tab extension 242, may project radially outward through a commissure window 170, and a wedge 148 may be disposed between the portions of the lateral tab portion 240 projecting radially outward from the commissure window 170, and more specifically, between the lateral outer portion 241. While not shown explicitly, a cloth (152) may be used to cover the wedge 148 and portions of the lateral tab portion 240. However, even when a cloth is used to further cover and assist in attachment of components of a commissure assemble 244, the cloth will not extend radially inward beyond the inner edges of the vertical tab extensions 242, so as to avoid potential contact between the moving portions of the leaflets 232 and the cloth, thereby minimizing or eliminating risk of leaflet abrasion.

Reference is now made to Figs. 8-9B, showing an exemplary embodiment of commissures assembly 344 formed from tabs 338 of leaflets 332. Fig. 8 shows an exemplary type of leaflet 332, which can be similar to leaflet 232, except when noted. The leaflet 332 has a rounded cusp edge 334, and a pair of generally oppositely-directed tabs 338 separating the cusp edge 334 and the free edge 336. Both tabs 338 include lateral tab portions 340, which extends in a lateral direction away from the cusp edge 234 and the free edge 236. Unlike leaflet 232, the leaflet 332 comprises only one vertical tab extension 342 extending vertically from one of the tabs 338. Similarly, unlike the case with most conventional leaflets, such as leaflet 132 shown in Fig. 3, the leaflet 332 is asymmetrical with respect to a leaflet central axis 20, since only one tab 138 on one side of the leaflet central axis 20 includes a vertical tab extension 342.

It will be clear that any reference to lateral tab portions (240, 340) extending in lateral directions away from the cusp edge, and any reference to a vertical tab extensions (242, 342), extending vertically from the lateral tab extensions, refer to flattened configurations of the leaflets (232, 332) as shown in Figs. 6 and 8, and that the lateral tab portions (240, 340) and/or the vertical tab extensions (242, 342) may assume other orientations once assembled within respective commissures (244, 344) and coupled to the frame (106).

Fig. 8 shows a leaflet 332 having two tabs 338aa and 338ab, provided with corresponding lateral tab portions 340aa and 340ab extending laterally from both sides thereof (at opposite directions). Both lateral tab portions 340 may have the same width W₂, which may be identical to the width W₁ of lateral tab portions 240. A vertical tab extension 342 extends from the lateral tab portion 340aa, preferably orthogonal thereto, while the opposite lateral tab portion 340ab is devoid of a vertical tab extension. The lateral tab portion 340aa comprises a lateral outer portion 341, defined as the sub-portion of the lateral tab portion 340aa projecting away from the vertical tab extension 342, and having a non-zero length.

While the vertical tab extension 342 is illustrated extending from the lateral tab portion 340aa, it will be clear that in other implementations, the vertical tab extension 342 can extend from the lateral tab portion 340ab, while the lateral tab portion 340aa is devoid of a vertical tab extension. While the vertical tab extension 342 is illustrated as extending upward, it will be clear that this is for illustrative purpose only, and that in other implementations, the vertical tab extension 342 may extend downward.

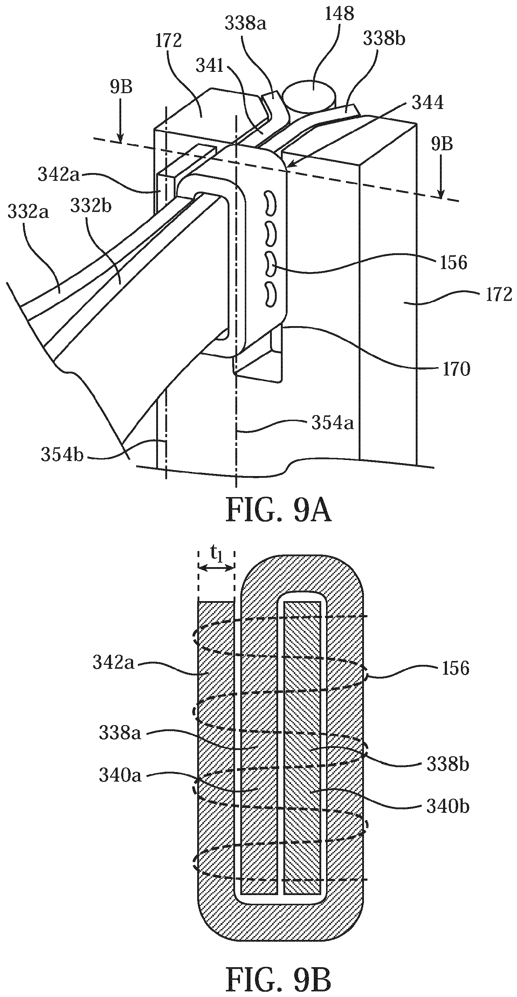

Fig. 9A shows an enlarged view of a commissure 344 mounted within a commissure window 170. Fig. 9B shows a cross-section view of the commissure 344 across plane 9B-9B of Fig. 9A. A commissure 344 is formed by attaching tabs 338 of adjacent leaflets 332 to each other, such that the vertical tab extension 342 of one tab 338 is folded over the tab 338 of the adjacent leaflet, which is devoid of a vertical tab extension 342. The vertical tab extension 342 can be long enough to be further folded over the lateral tab portion 340 it is extending from.

A suture 156 can be used to couple the adjacent lateral tab portions 340 and the vertical tab extension 342 folded thereover, to form a four-layered structure as shown in Fig. 9B, extending from the inner surface of the window frame portion 112. The resulting four layered structure is more resistant to bending, or articulating, than the portions of the leaflets 332 that are radially inward of the tabs 338, and serves to offset the articulation axes 354 radially inward, away from the frame 106. By having the leaflets 332 flex against the inner edges of the vertical tab extension 342, contact between the moving portions of the leaflets 332 (especially the free edges 336 of the leaflets) and the frame 106 can be avoided during working cycles, which in turn improves the durability of the valve.

Advantageously, since the vertical tab extension 342 constitutes an integral portion of the leaflet 232, the moving portions of the leaflets 232 articulate about portions of the leaflets 332 made of the same material (e.g., bovine pericardium), instead of articulating about external components such as reinforcement members (150) or cloth (152), which can comprise other materials (e.g., textiles, polymeric materials, and the like). The absence of such materials can significantly reduce abrasion and wear of the leaflet material.

The vertical tab extension 342 may have a length L₂ (see Fig. 8), and the tabs 338 may have a thickness t₁ (see Figs. 9B). According to some embodiments, the length L₂ of the vertical tab extension 342 is greater than the length L₁ of the vertical tab extension 242. According to some embodiments, the length L₂ of the vertical tab extension 342 is at least as great as the width W₂ of the lateral tab portion 340. According to some embodiments, the length L₂ of the vertical tab extension 342 is at least as great as 200% of the width W₁ of the lateral tab portion 340. According to some embodiments, the length L₂ of the vertical tab extension 342 is at least as great as the sum of 200% of the width W₁ of the lateral tab portion 340, and 200% of the thickness t₁ of the tab 238.

As shown in Figs. 9A-9B, two adjacent tabs 338a and 338b of respective leaflets 332a and 332b, may be secured to each other such that the vertical tab extension 342 is wrapped around both lateral tab portions 340a and 340b of the commissure 344. More specifically, the vertical tab extension 342 may be folded over the adjacent lateral tab portion 340b, and then further folded backward over the lateral tab portion 340a it extends from.

Once in the folded position shown in Figs. 9A-9B, a suture 156 may be used to stitch all four layers, including both folded portions of the vertical tab extension 342, the lateral tab portion 340b, the lateral tab portion 340a, together. It is noted that suture 156 is used by way of illustration and not limitation, and that other securement means may be used to attach the four layers to each other. Moreover, more than one suture 156 may be used, and while shown to pass through the layers in an in-and-out pattern, any other stitching pattern is contemplated.

As shown in Fig. 9A, the folded vertical tab extension 342 of the commissure assembly 344 is positioned radially inward with respect to the frame 106. The remaining portions of the lateral tab portions 340 extending beyond the vertical tab extension 242, including the lateral outer portion 341, may project radially outward through a commissure window 170, and a wedge 148 may be disposed between the portions of the lateral tab portion 340 at the radially outer side of the commissure window 170. While not shown explicitly, a cloth (152) may be used to cover the wedge 148 and portions of the lateral tab portion 340. However, even when a cloth is used to further cover and assist in attachment of components of a commissure assemble 344, the cloth will not extend radially inward beyond the inner edges of the vertical tab extension 342, so as to avoid potential contact between the moving portions of the leaflets 332 and the cloth, thereby minimizing or eliminating risk of leaflet abrasion.

It will be appreciated that placement of the commissure assemblies 244 and 344 within commissure windows 170 of axially extending window frame portions 112, as shown throughout the Figures, is show by way of illustration and not limitation, and that commissure assemblies 244, 344 comprising tabs 238, 338 with at least one vertical tab extension 242, 342, secured to each other, may be used in combination with any other commissure attachment configuration known in the art, to secure commissures to a frame of a prosthetic valve. For example, the lateral tab portion 240, 340, including lateral outer portions 241, 341 thereof, can be folded over or wrapped around vertical strut portions of a frame, and/or around commissure posts or rods attached to a frame. Similarly, commissure windows may be formed not only within integral structures of the frame, such as axially extending window frame portions (112), but may be similarly formed by clip members attached to the frame, and/or within outflow portions of expansion and locking assemblies of mechanically expandable valves.

## Claims

1. A leaflet assembly (130) comprising a plurality of leaflets (132; 232, 332) configured to regulate flow through a prosthetic heart valve (100), wherein each leaflet (132; 232; 332) comprises a pair of oppositely-directed tabs (138; 238; 338), wherein each of the tabs (138; 238; 338) comprises a lateral tab portion (240; 340), and wherein both of the tabs (138; 238; 338) of each leaflet (132; 232; 332) comprise a vertical tab extension (242; 342) extending at a non-zero angle from the lateral tab portion (240; 340);
wherein each leaflet (132; 232; 332) is asymmetrical with respect to a leaflet central axis (20) thereof; and
wherein the vertical tab extension (242; 342) of each of the tabs (138; 238, 338) of each leaflet (132; 232; 332) is folded over a lateral tab portion (240; 340) of an adjacent leaflet (132; 232; 332) to form a plurality of annularly spaced commissure assemblies (144; 244; 344) of the leaflet assembly (130).

2. The leaflet assembly (130) of claim 1, wherein each vertical tab extension (242; 342) is orthogonal with respect to the lateral tab portion (240; 340) it extends from.

3. The leaflet assembly (130) of claim 1 or 2, wherein the vertical tab extension (242; 342) of each of the tabs (138; 238; 338) of each leaflet (132; 232; 332) extend from the lateral tab portions (240; 340) in opposite vertical directions with respect to each other.

4. The leaflet assembly (130) of claim 3, wherein each vertical tab extension (242; 342) of two adjacent vertical tab extensions (242; 342) within each commissure assembly (144; 244; 344), is folded over a lateral tab portion (240; 340) of an adjacent leaflet (132; 232; 332) of the same commissure assembly (144; 244; 344).

5. A prosthetic heart valve (100) comprising a leaflet assembly (130) according to one of claims 1 to 4; and
a frame (106) movable between a radially compressed configuration and a radially expanded configuration; wherein the leaflet assembly (130) is mounted within the frame (106).

6. The prosthetic heart valve (100) of claim 5, wherein the vertical tab extensions (242; 342) of all commissure assemblies (144; 244; 344) are positioned radially inward with respect to the frame (106).

7. The prosthetic heart valve (100) of claim 5 or 6, further comprising an inner skirt (122) attached to the frame (106).

8. The prosthetic heart valve (100) of any one of claims 5 to 7, wherein the prosthetic valve is balloon-expandable.

9. The prosthetic heart valve (100) of any one of claims 5 to 7, wherein the prosthetic valve is self-expandable or mechanically expandable.

10. The prosthetic heart valve (100) of any one of claims 5 to 7, wherein the frame (106) is made of one of stainless steel, a nickel-based alloy, polymers or combinations thereof.

11. A method of assembling a leaflet assembly (130) configured to regulate flow through a prosthetic heart valve (100), comprising the steps of:
providing a plurality of asymmetrical leaflets (132; 232; 332), wherein each leaflet (132; 232; 332) comprises a pair of oppositely-directed tabs (138; 238; 338), wherein each of the tabs (138; 238; 338) comprises a lateral tab portion (240; 340), and wherein each of the tabs (138; 238; 338) of each leaflet (132; 232; 332) comprises a vertical tab extension (242; 342) extending at a non-zero angle, from the lateral tab portion (240; 340); and
folding the vertical tab extension (242; 342) of each of the tabs of each of the leaflets (132; 232; 332) over a lateral tab portion (240; 340) of an adjacent leaflet (132; 232; 332) to form a plurality of annularly spaced commissure assemblies (144; 244; 344) of the leaflet assembly (130).

12. The method of claim 11, wherein each vertical tab extension (242; 342) is orthogonal with respect to the lateral tab portion (240; 340) it extends from.

13. The method of claim 11 or 12, wherein the vertical tab extension (242; 342) of each of the tabs (138; 238; 338) of each leaflet (132; 232; 332) extend from both lateral tab portions (240; 340) in opposite vertical directions with respect to each other.

14. The method of claim 13, wherein the step of folding vertical tab extension (242; 342) of one of the leaflets (132; 232; 332) over a lateral tab portion (240; 340) of an adjacent leaflet (132; 232; 332), comprises folding each vertical tab extension (242; 342) of two adjacent vertical tab extensions (242; 342) within each commissure assembly (144; 244; 344), over a lateral tab portion (240; 340) of an adjacent leaflet (132; 232; 332) of the same commissure assembly (144; 244; 344).

15. The method of any one of claims 11 to 14, further comprising a step of mounting the leaflet assembly (130) within a frame (106) of a prosthetic heart valve (100).

## Patentansprüche

1. Segelanordnung (130), umfassend eine Mehrzahl von Segeln (132; 232, 332), die dazu ausgelegt sind, den Durchfluss durch eine Herzklappenprothese (100) zu regulieren, wobei jedes Segel (132; 232; 332) ein Paar entgegengesetzt gerichteter Laschen (138; 238; 338) umfasst, wobei jede der Laschen (138; 238; 338) einen lateralen Laschenabschnitt (240; 340) umfasst, und wobei beide der Laschen (138; 238; 338) jedes Segels (132; 232; 332) einen senkrechten Laschenfortsatz (242; 342) aufweisen, der sich in einem von Null verschiedenen Winkel von dem lateralen Laschenabschnitt (240; 340) aus erstreckt;
wobei jedes Segel (132; 232; 332) bezüglich einer Segelmittelachse (20) asymmetrisch ist; und
wobei der senkrechte Laschenfortsatz (242; 342) jeder der Laschen (138; 238; 338) jedes Segels (132; 232; 332) über einen lateralen Laschenabschnitt (240; 340) eines angrenzenden Segels (132; 232; 332) gefaltet ist, um eine Mehrzahl von in Umfangsrichtung beabstandeten Kommissuranordnungen (144; 244; 344) der Segelanordnung (130) zu bilden.

2. Segelanordnung (130) gemäß Anspruch 1, wobei jeder senkrechte Laschenfortsatz (242; 342) orthogonal zu dem lateralen Laschenabschnitt (240; 340) ist, von dem er sich aus erstreckt.

3. Segelanordnung (130) gemäß Anspruch 1 oder 2, wobei sich der senkrechte Laschenfortsatz (242; 342) jeder der Laschen (138; 238; 338) jedes Segels (132; 232; 332) von den lateralen Laschenabschnitten (240; 340) in entgegengesetzten vertikalen Richtungen relativ zueinander erstreckt.

4. Segelanordnung (130) gemäß Anspruch 3, wobei jeder senkrechte Laschenfortsatz (242; 342) von zwei benachbarten senkrechten Laschenfortsätzen (242; 342) innerhalb jeder Kommissuranordnung (144; 244; 344) über einen lateralen Laschenabschnitt (240; 340) eines angrenzenden Segels (132; 232; 332) derselben Kommissuranordnung (144; 244; 344) gefaltet ist.

5. Herzklappenprothese (100), umfassend eine Segelanordnung (130) gemäß einem der Ansprüche 1 bis 4; und
einen Rahmen (106), der zwischen einer radial komprimierten Konfiguration und einer radial expandierten Konfiguration bewegbar ist; wobei die Segelanordnung (130) innerhalb des Rahmens (106) montiert ist.

6. Herzklappenprothese (100) gemäß Anspruch 5, wobei die senkrechten Laschenfortsätze (242; 342) aller Kommissuranordnungen (144; 244; 344) radial nach innen in Bezug auf den Rahmen (106) positioniert sind.

7. Herzklappenprothese (100) gemäß Anspruch 5 oder 6, ferner umfassend eine Innenmanschette (122), die an dem Rahmen (106) befestigt ist.

8. Herzklappenprothese (100) gemäß einem der Ansprüche 5 bis 7, wobei die Herzklappenprothese ballonexpandierbar ist.

9. Herzklappenprothese (100) gemäß einem der Ansprüche 5 bis 7, wobei die Herzklappenprothese selbstexpandierbar oder mechanisch expandierbar ist.

10. Herzklappenprothese (100) gemäß einem der Ansprüche 5 bis 7, wobei der Rahmen (106) aus rostfreiem Stahl, einer nickelbasierten Legierung, Polymeren oder Kombinationen davon hergestellt ist.

11. Verfahren zum Zusammensetzen einer Segelanordnung (130), die dazu ausgelegt ist, den Durchfluss durch eine Herzklappenprothese (100) zu regulieren, umfassend die Schritte:
Bereitstellen einer Mehrzahl asymmetrischer Segel (132; 232; 332), wobei jedes Segel (132; 232; 332) ein Paar entgegengesetzt gerichteter Laschen (138; 238; 338) umfasst, wobei jede der Laschen (138; 238; 338) einen lateralen Laschenabschnitt (240; 340) umfasst, und wobei jede der Laschen (138; 238; 338) jedes Segels (132; 232; 332) einen senkrechten Laschenfortsatz (242; 342) aufweist, der sich in einem von Null verschiedenen Winkel von dem lateralen Laschenabschnitt (240; 340) aus erstreckt; und
Falten des senkrechten Laschenfortsatzes (242; 342) jeder der Laschen jedes der Segel (132; 232; 332) über einen lateralen Laschenabschnitt (240; 340) eines angrenzenden Segels (132; 232; 332) um eine Mehrzahl von in Umfangsrichtung beabstandeten Kommissuranordnungen (144; 244; 344) der Segelanordnung (130) zu bilden.

12. Verfahren gemäß Anspruch 11, wobei jeder senkrechte Laschenfortsatz (242; 342) orthogonal zu dem lateralen Laschenabschnitt (240; 340) ist, von dem er sich aus erstreckt.

13. Verfahren gemäß Anspruch 11 oder 12, wobei sich der senkrechte Laschenfortsatz (242; 342) jeder der Laschen (138; 238; 338) jedes Segels (132; 232; 332) von beiden lateralen Laschenabschnitten (240; 340) in entgegengesetzten vertikalen Richtungen relativ zueinander erstreckt.

14. Verfahren gemäß Anspruch 13, wobei der Schritt des Faltens des senkrechten Laschenfortsatzes (242; 342) eines der Segel (132; 232; 332) über einen lateralen Laschenabschnitt (240; 340) eines angrenzenden Segels (132; 232; 332) das Falten jedes senkrechten Laschenfortsatzes (242; 342) von zwei benachbarten senkrechten Laschenfortsätzen (242; 342) innerhalb jeder Kommissuranordnung (144; 244; 344) über einen lateralen Laschenabschnitt (240; 340) eines angrenzenden Segels (132; 232; 332) derselben Kommissuranordnung (144; 244; 344) umfasst.

15. Verfahren gemäß einem der Ansprüche 11 bis 14, ferner umfassend einen Schritt des Montierens der Segelanordnung (130) innerhalb eines Rahmens (106) einer Herzklappenprothese (100).

## Revendications

1. Ensemble de feuillets (130) comprenant une pluralité de feuillets (132 ; 232, 332) conçu pour réguler l'écoulement à travers une valvule cardiaque prothétique (100), chaque feuillet (132 ; 232 ; 332) comprenant une paire de languettes (138 ; 238 ; 338) dirigées de manière opposée, chacune des languettes (138 ; 238 ; 338) comprenant une partie languette latérale (240 ; 340) et les deux languettes (138 ; 238 ; 338) de chaque feuillet (132 ; 232 ; 332) comprenant une extension verticale de languette (242 ; 342) s'étendant selon un angle non nul à partir de la partie languette latérale (240 ; 340) ;
chaque feuillet (132 ; 232 ; 332) étant asymétrique par rapport à un axe central de feuillet (20) correspondant ; et
l'extension verticale de languette (242 ; 342) de chacune des languettes (138 ; 238, 338) de chaque feuillet (132 ; 232 ; 332) étant pliée sur une partie languette latérale (240 ; 340) d'un feuillet adjacent (132 ; 232 ; 332) afin de former une pluralité d'ensembles commissure espacés de manière annulaire (144 ; 244 ; 344) de l'ensemble de feuillets (130).

2. Ensemble de feuillets (130) selon la revendication 1, chaque extension verticale de languette (242 ; 342) étant orthogonale par rapport à la partie languette latérale (240 ; 340) à partir de laquelle elle s'étend.

3. Ensemble de feuillets (130) selon la revendication 1 ou 2, l'extension verticale de languette (242 ; 342) de chacune des languettes (138 ; 238 ; 338) de chaque feuillet (132 ; 232 ; 332) s'étendant à partir des parties languette latérale (240 ; 340) dans des sens verticaux opposés l'un par rapport à l'autre.

4. Ensemble de feuillets (130) selon la revendication 3, chaque extension verticale de languette (242 ; 342) de deux extensions verticales de languette (242 ; 342) adjacentes à l'intérieur de chaque ensemble commissure (144 ; 244 ; 344) étant pliée sur une partie languette latérale (240 ; 340) d'un feuillet adjacent (132 ; 232 ; 332) du même ensemble commissure (144 ; 244 ; 344).

5. Valvule cardiaque prothétique (100) comprenant un ensemble de feuillets (130) selon l'une des revendications 1 à 4 ; et
un cadre (106) mobile entre une configuration radialement comprimée et une configuration radialement déployée ; l'ensemble de feuillets (130) étant monté à l'intérieur du cadre (106).

6. Valvule cardiaque prothétique (100) selon la revendication 5, les extensions verticales de languette (242 ; 342) de tous les ensembles commissure (144 ; 244 ; 344) étant positionnées radialement vers l'intérieur par rapport au cadre (106).

7. Valvule cardiaque prothétique (100) selon la revendication 5 ou 6, comprenant en outre une collerette interne (122) fixée au cadre (106).

8. Valvule cardiaque prothétique (100) selon l'une quelconque des revendications 5 à 7, la valvule prothétique étant déployable par ballonnet.

9. Valvule cardiaque prothétique (100) selon l'une quelconque des revendications 5 à 7, la valvule prothétique étant autodéployable ou mécaniquement déployable.

10. Valvule cardiaque prothétique (100) selon l'une quelconque des revendications 5 à 7, le cadre (106) étant fabriqué en acier inoxydable, en alliage à base de nickel, en polymères ou en combinaisons de ceux-ci.

11. Procédé d'assemblage d'un ensemble de feuillets (130) conçu pour réguler l'écoulement à travers une valvule cardiaque prothétique (100), comprenant les étapes de :
fourniture d'une pluralité de feuillets asymétriques (132 ; 232 ; 332), chaque feuillet (132 ; 232 ; 332) comprenant une paire de languettes dirigées de manière opposée (138 ; 238 ; 338), chacune des languettes (138 ; 238 ; 338) comprenant une partie languette latérale (240 ; 340) et chacune des languettes (138 ; 238 ; 338) de chaque feuillet (132 ; 232 ; 332) comprenant une extension verticale de languette (242 ; 342) s'étendant selon un angle non nul à partir de la partie languette latérale (240 ; 340) ; et
pliage de l'extension verticale de languette (242 ; 342) de chacune des languettes de chacun des feuillets (132 ; 232 ; 332) sur une partie languette latérale (240 ; 340) d'un feuillet adjacent (132 ; 232 ; 332) afin de former une pluralité d'ensembles commissure espacés de manière annulaire (144 ; 244 ; 344) de l'ensemble de feuillets (130).

12. Procédé selon la revendication 11, chaque extension verticale de languette (242 ; 342) étant orthogonale par rapport à la partie languette latérale (240 ; 340) à partir de laquelle elle s'étend.

13. Procédé selon la revendication 11 ou 12, l'extension verticale de languette (242 ; 342) de chacune des languettes (138 ; 238 ; 338) de chaque feuillet (132 ; 232 ; 332) s'étendant à partir des deux parties languette latérale (240 ; 340) dans des sens verticaux opposés l'un par rapport à l'autre.

14. Procédé selon la revendication 13, l'étape de pliage de l'extension verticale de languette (242 ; 342) de l'un des feuillets (132 ; 232 ; 332) sur une partie languette latérale (240 ; 340) d'un feuillet adjacent (132 ; 232 ; 332), comprenant le pliage de chaque extension verticale de languette (242 ; 342) de deux extensions verticales de languette adjacentes (242 ; 342) à l'intérieur de chaque ensemble commissure (144 ; 244 ; 344), sur une partie languette latérale (240 ; 340) d'un feuillet adjacent (132 ; 232 ; 332) du même ensemble commissure (144 ; 244 ; 344).

15. Procédé selon l'une quelconque des revendications 11 à 14, comprenant en outre une étape de montage de l'ensemble de feuillets (130) à l'intérieur d'un cadre (106) d'une valvule cardiaque prothétique (100).
